# EUROPÄISCHE PATENTSCHRIFT

(11) **EP 3 158 965 B1**
(45) Veröffentlichungstag und Bekanntmachung des Hinweises auf die Patenterteilung: **08.11.2017**
(21) Anmeldenummer: 16193872.5
(22) Anmeldetag: 14.10.2016
(51) Int. Cl.: A61B 90/70, A61B 17/29

(54) **MEDIZINISCHES INSTRUMENT**
MEDICAL INSTRUMENT
INSTRUMENT MÉDICAL

(30) Priorität: 19.10.2015 DE 102015117731
(43) Veröffentlichungstag der Anmeldung: 26.04.2017
(73) Patentinhaber: Karl Storz GmbH & Co. KG, 78532 Tuttlingen (DE)
(72) Erfinder: Thouèment, Yann, 78690 Les Essarts le roi (FR); Besse, Régis, 78610 Le Perray en Yvelines (FR)

(56) Entgegenhaltungen:
- EP-A1- 2 095 778
- EP-A1- 2 581 059
- EP-A1- 2 837 354
- WO-A1-2010/105649

## Beschreibung

Die vorliegende Erfindung betrifft ein medizinisches Instrument nach dem Oberbegriff von Anspruch 1.

Derartige medizinische Instrumente sind bekannt. Insbesondere ist aus der Europäischen Patentanmeldung EP 2 837 354 A1 ein medizinischer Manipulator bekannt mit einem Schaft, einem an dessen Spitze angeordneten Arbeitsabschnitt mit einem Greifer und einer Handhabe, mit der der Greifer betätigt werden kann. Der Arbeitsabschnitt weist einen Biegeabschnitt auf zwischen dem Greifer und dem Schaft, der eine Abwinkelung des Greifers aus einer axialen Richtung des Schafts aufgrund einer von der Handhabe her übertragenen Biegebetätigungskraft bewirkt. Innerhalb eines Außenrohrs des Schafts verlaufen ein erstes und ein zweites Halbrohr, die jeweils als entlang seiner Längsachse geteiltes Hohlrohr ausgebildet sind und die mit ihren geöffneten Seiten einander zugewandt parallel zueinander angeordnet sind und die in Längsrichtung des Schafts verschiebbar sind. Das erste und das zweite Halbrohr sind mit auf einander gegenüberliegenden Seiten des Biegeabschnitts geführten Schiebeelementen verbunden. Durch eine Verschiebung der Halbrohre in Schaftlängsrichtung gegeneinander kann über eine entsprechende Verschiebung der Schiebelemente eine Biegung des Biegeabschnitts bewirkt werden. Innerhalb der beiden Halbrohre erstreckt sich ein Innenrohr, durch dessen Rotation um die Längsachse des Schafts eine Rotation des Greifers um eine Längsachse des Greifers sowie durch dessen Verschiebung in Längsrichtung des Schafts ein Öffnen oder Schließen des Greifers bewirkt werden kann. Das Innenrohr erstreckt sich in den Biegeabschnitt und folgt einer Biegung des Biegeabschnitts. Innerhalb des Innenrohrs verlaufen elektrische Leitungen, mittels derer die Greifelemente des Greifers mit einer Hochfrequenz-Spannungsquelle verbunden sind. Die Handhabe umfasst einen Handgriff, mit dem ein Benutzer den medizinischen Manipulator halten kann sowie Bedienelemente zur Steuerung der genannten Funktionen des Arbeitsabschnitts.

Bei medizinischen Instrumenten, insbesondere bei wiederverwendbaren medizinischen Instrumenten, ist es erforderlich, zumindest solche Teile des Instruments, die mit Körperflüssigkeiten in Kontakt kommen können, reinigen und/oder sterilisieren zu können. Bei endoskopischen medizinischen Instrumenten gilt dies zumindest für diejenigen Teile des Instruments, die zur Einführung in einen körperinneren Hohlraum eines menschlichen oder tierischen Körpers bestimmt sind. Hierfür kommt beispielsweise eine Reinigung mit einer Reinigungsflüssigkeit bzw. eine Sterilisation mit einem flüssigen oder gasförmigen Sterilisationsfluid in Frage.

Es ist Aufgabe der vorliegenden Erfindung, ein gattungsgemäßes medizinisches Instrument anzugeben, das hinsichtlich seiner Reinigungs- und/oder Sterilisationsmöglichkeiten verbessert ist.

Diese Aufgabe wird durch ein medizinisches Instrument gemäß Anspruch 1 sowie durch ein medizinisches Instrument gemäß Anspruch 14 gelöst.

Vorteilhafte Weiterbildungen der Erfindung ergeben sich aus den Unteransprüchen.

Ein erfindungsgemäßes medizinisches Instrument weist einen langerstreckten, vorzugsweise starr ausgebildeten Schaft auf. Das medizinische Instrument ist insbesondere als endoskopisches Instrument ausgebildet, wobei der Schaft zumindest abschnittsweise zur Einführung in einen körperinneren Hohlraum eines menschlichen oder tierischen Körpers ausgebildet ist. Das medizinische Instrument kann beispielsweise ein Manipulator sein, mit dem bei einem endoskopischen Eingriff chirurgische Manipulationen in dem körperinneren Hohlraum vorgenommen werden können.

Weiter umfasst das medizinische Instrument eine am distalen, d. h. benutzerfernen Ende des Schafts angeordnete Werkzeuganordnung, die insbesondere zur Durchführung chirurgischer Manipulationen in dem körperinneren Hohlraum ausgebildet ist. Weiter umfasst das medizinische Instrument eine an einem proximalen, d. h. benutzernahen Ende des Schafts angeordnete Handhabe, die zur Betätigung der Werkzeuganordnung durch einen Benutzer dient und die hierfür ein oder mehrere Bedienelemente sowie einen Handgriff aufweisen kann.

Der Schaft des erfindungsgemäßen medizinischen Instruments weist ein langerstrecktes Außenrohr auf, das im wesentlichen hohlzylindrisch ausgebildet ist und dessen Länge und Außendurchmesser insbesondere derart gewählt sind, dass es zumindest mit einem distalen Abschnitt in den körperinneren Hohlraum des menschlichen oder tierischen Körpers einführbar ist. Der distale Abschnitt des Schafts und die Werkzeuganordnung können somit in den körperinneren Hohlraum eingeführt werden. Innerhalb des Außenrohrs, d. h. in dem durch das im Wesentlichen hohlzylindrische Außenrohr gebildeten längs erstreckten Hohlraum, ist ein erstes Übertragungselement angeordnet, das zur Übertragung einer Betätigungsbewegung von der Handhabe zur Werkzeuganordnung zur Betätigung mindestens eines ersten Funktionselements der Werkzeuganordnung ausgebildet ist. Das erste Funktionselement umfasst insbesondere mindestens ein bewegliches Bauteil, das mittels des ersten Übertragungselements bewegt werden kann. Das erste Übertragungselement ist hierfür distalseitig mit dem mindestens einen ersten Funktionselement der Werkzeuganordnung und proximalseitig mit einer entsprechenden Betätigungseinrichtung der Handhabe direkt oder indirekt verbunden. Das erste Übertragungselement kann ebenfalls im Wesentlichen hohlzylindrisch ausgebildet sein.

Das erste Übertragungselement umfasst ein erstes und ein zweites Halbrohr, die jeweils einen bogenförmigen, vorzugsweise einen etwa halbkreisförmigen Querschnitt aufweisen. Das erste und das zweite Halbrohr stellen insbesondere die zwei Teile eines in seiner Längsrichtung entlang einer Längsmittelebene geschnittenen zylinderförmigen Hohlrohrs dar. Das erste und das zweite Halbrohr sind mit ihren jeweiligen längsseitigen Öffnungen einander zugewandt angeordnet, so dass sie insgesamt näherungsweise einen Hohlzylinder bilden. Dabei stehen die Längsschnittflächen des ersten und des zweiten Halbrohrs einander gegenüber und können zwischen sich jeweils einen Spalt bilden. Der durch das erste und das zweite Halbrohr gebildete Hohlzylinder ist vorzugsweise koaxial innerhalb des Außenrohrs des Schafts angeordnet, so dass die Mittellängsachse des durch das erste und das zweite Halbrohr gebildeten Hohlzylinders näherungsweise mit der Mittellängsachse des Außenrohrs zusammenfällt. Insbesondere können das erste und das zweite Halbrohr von der Mittellängsachse aus gesehen jeweils einen Bogen von näherungsweise 180 ° bzw. in dem Fall, dass zwischen dem ersten und dem zweiten Halbrohr ein Spalt vorgesehen ist, etwas weniger als 180 ° einnehmen. Das erste und das zweite Halbrohr sind relativ zum Außenrohr verschiebbar gelagert und zur Betätigung des ersten Funktionselements der Werkzeuganordnung relativ zueinander verschiebbar.

Weiter ist in innerhalb des ersten Übertragungselements, d. h. in einem von dem ersten Halbrohr und dem zweiten Halbrohr gebildeten längs erstreckten Hohlraum, ein zweites Übertragungselement angeordnet, das zur Übertragung einer Betätigungsbewegung von der Handhabe zur Werkzeuganordnung zur Betätigung mindestens eines zweiten Funktionselements der Werkzeuganordnung ausgebildet ist. Das zweite Funktionselement umfasst insbesondere mindestens ein bewegliches Bauteil, das mittels des zweiten Übertragungselements bewegt werden kann. Hierfür ist das zweite Übertragungselement distalseitig mit dem zweiten Funktionselement und proximalseitig mit einer entsprechenden Betätigungseinrichtung der Handhabe direkt oder indirekt verbunden.

Erfindungsgemäß ist in einem proximalen Endabschnitt des Schafts ein gegenüber dem Außenrohr erweitertes Schaftgehäuse vorgesehen, das fluiddicht mit dem Außenrohr, insbesondere einem proximalen Ende des Außenrohrs, verbunden ist. Der Schaft des erfindungsgemäßen medizinischen Instruments umfasst somit mindestens einen distalen Abschnitt, in dem das Außenrohr eine radiale Außenfläche des Schafts bildet und der insbesondere zur Einführung in einen körperinneren Hohlraum geeignet ist, und einen proximalen Endabschnitt, dessen radiale Außenfläche gegenüber dem mindestens einen distalen Abschnitt erweitert ist und beispielsweise zylindrisch mit einem gegenüber diesem größeren Außendurchmesser ausgebildet ist. Das Schaftgehäuse ist insbesondere zwischen dem distalen Abschnitt des Schafts und der Handhabe angeordnet. Das Außenrohr kann sich abschnittsweise in das Schaftgehäuse hinein erstrecken. Weiterhin ist zumindest abschnittsweise innerhalb des Schaftgehäuses ein im Wesentlichen zylindrisch ausgebildetes, umfänglich geschlossenes erstes Hohlrohr angeordnet, das mit dem ersten Halbrohr, insbesondere mit einem proximalen Ende des ersten Halbrohrs, verbunden ist. Zumindest abschnittsweise innerhalb des ersten Hohlrohrs ist ein ebenfalls im Wesentlichen zylindrisch ausgebildetes und umfänglich geschlossenes zweites Hohlrohr angeordnet, das mit dem zweiten Halbrohr, insbesondere mit einem proximalen Ende des zweiten Halbrohrs, verbunden ist. Das erste Hohlrohr ist gemeinsam mit dem ersten Halbrohr und das zweite Hohlrohr gemeinsam mit dem zweiten Halbrohr in Längsrichtung relativ zum Schaftgehäuse verschiebbar.

Weiter ist erfindungsgemäß zwischen einer vorzugsweise zylindrischen Innenfläche des Schaftgehäuses und einer vorzugsweise zylindrischen Außenfläche des ersten Hohlrohrs eine erste, im Wesentlichen ringförmig ausgebildete Dichtung angeordnet sowie zwischen einer Innenfläche des ersten Hohlrohrs und einer Außenfläche des zweiten Hohlrohrs eine zweite, ebenfalls im Wesentlichen ringförmige Dichtung.

Dadurch, dass das erste Halbrohr proximalseitig mit einem ersten Hohlrohr verbunden ist und dass das zweite Halbrohr proximalseitig mit einem zumindest abschnittsweise innerhalb des ersten Hohlrohrs angeordneten zweiten Hohlrohr verbunden ist, sowie dass zwischen dem mit dem Außenrohr verbundenen Schaftgehäuse und dem ersten Hohlrohr und zwischen dem ersten und dem zweiten Hohlrohr jeweils eine im Wesentlichen ringförmige Dichtung angeordnet ist, wird es ermöglicht, in einen zwischen dem Außenrohr und dem ersten und dem zweiten Halbrohr gebildeten Hohlraum eine Reinigungsflüssigkeit oder ein Sterilisationsfluid einzuleiten, ohne dass diese bzw. dieses proximalseitig aus dem Schaft austreten kann. Hierdurch wird die Möglichkeit geschaffen, die durch das Außenrohr und das erste und das zweite Übertragungselement gebildete Anordnung mit einer Reinigungsflüssigkeit oder einem Sterilisationsfluid zu spülen, ohne dass diese bzw. dieses in die proximalseitig mit dem Schaft verbundene Handhabe gelangen kann. Auf diese Weise kann eine zumindest teilweise Reinigung und/oder Sterilisation des Inneren des Schafts ermöglicht werden, wobei gleichzeitig die Bauelemente der Handhabe vor dem Reinigungs- bzw. Sterilisationsfluid geschützt werden können, beispielsweise elektrische oder elektronische Baugruppen, die durch eine Reinigungsflüssigkeit bzw. ein Sterilisationsfluid beschädigt werden können. Ferner kann hierdurch bei einem mit dem medizinischen Instrument durchgeführten endoskopischen Eingriff eine Kontamination der Handhabe mit Körperflüssigkeiten, Spülflüssigkeit und/oder Insufflationsgas verhindert werden.

Vorzugsweise weist das Außenrohr und / oder das Schaftgehäuse eine Spülöffnung auf, durch die eine Reinigungsflüssigkeit oder ein Sterilisationsfluid in das Außenrohr bzw. in das Schaftgehäuse einleitbar ist oder aus diesem austreten kann. Zum Schutz der Handhabe vor Beschädigung oder Verschmutzung ist in diesem Fall die erste Dichtung proximalseitig der Spülöffnung angeordnet. Die zweite Dichtung ist vorzugsweise ebenfalls proximalseitig der Spülöffnung angeordnet. Zumindest kann die erste sowie vorzugsweise auch die zweite Dichtung durch Verschieben des ersten bzw. des zweiten Hohlrohrs in eine jeweilige Position gebracht werden, in die erste bzw. die erste und die zweite Dichtung proximalseitig der Spülöffnung angeordnet sind. Hierdurch wird es beispielsweise ermöglicht, proximalseitig eine Reinigungsflüssigkeit oder ein Sterilisationsfluid in den Schaft einzuleiten, die distalseitig aus diesem austreten kann. Dadurch wird die Reinigung bzw. Sterilisation des erfindungsgemäßen Instruments erleichtert.

In bevorzugter Weise sind die erste und die zweite Dichtung derart ausgebildet, dass über einen gesamten Verschiebeweg des ersten und des zweiten Halbrohrs bzw. des ersten und des zweiten Hohlrohrs eine Dichtwirkung erzielbar ist. Hierfür können die erste und die zweite Dichtung jeweils zum Gleiten auf einer zylindrischen Gegenfläche ausgebildet sein und bei einer Verschiebung des ersten Hohlrohrs gegenüber dem zweiten Hohlrohr sowie einer Verschiebung des ersten Hohlrohrs gegenüber dem Schaftgehäuse auf einer jeweiligen Gegenfläche gleiten. Hierdurch wird eine bei jeder Verschiebeposition des ersten und des zweiten Halbrohrs wirksame Abdichtung und somit eine von einer Betätigung bzw. einer Stellung des ersten Funktionselements unabhängige Dichtwirkung ermöglicht.

Gemäß einer bevorzugten Ausführungsform der Erfindung sind die erste und die zweite Dichtung mit dem ersten Hohlrohr verbunden. Insbesondere ist die erste Dichtung auf einer Außenfläche und die zweite Dichtung auf einer Innenfläche des ersten Hohlrohrs befestigt. Hierfür kann beispielweise die im Wesentlichen ringförmig ausgebildete erste Dichtung in eine umlaufende Nut bzw. eine ringförmige Ausnehmung der Außenfläche des ersten Hohlrohrs und die ebenfalls im Wesentlichen ringförmige zweite Dichtung in eine umlaufende Nut bzw. Ausnehmung der Innenfläche des ersten Hohlrohrs eingesetzt sein. Die Innenfläche des Schaftgehäuses kann in diesem Fall im Wesentlichen zylindrisch ausgebildet sein und als Gegenfläche dienen, auf der die erste Dichtung bei einer Verschiebung des ersten Hohlrohrs gegenüber dem Schaftgehäuse dichtend gleitet. Ferner kann in diesem Fall die Außenfläche des zweiten Hohlrohrs abschnittsweise im Wesentlichen zylindrisch ausgebildet sein und als Gegenfläche dienen, auf der die zweite Dichtung bei einer Verschiebung des zweiten Hohlrohrs gegenüber dem ersten Hohlrohr dichtend gleitet. Hierdurch wird eine besonders einfache Ausführungsform mit einer sicheren Abdichtung ermöglicht.

Weiterhin ist es bevorzugt, dass das erste und das zweite Hohlrohr proximalseitig aus dem Schaftgehäuse in die Handhabe hineinreichen. Hierfür können das erste und das zweite Hohlrohr proximalseitig aus dem Schaftgehäuse herausragen, so dass diese bis in die Handhabe hineinreichen, wobei die Handhabe proximalseitig an das Schaftgehäuse angesetzt sein kann. Es kann auch vorgesehen sein, dass das Schaftgehäuse über ein Verbindungsrohr mit der Handhabe verbunden ist, wobei das erste und das zweite Hohlrohr über ein proximales Ende des Verbindungsrohrs hinausragen, so dass das erste und das zweite Hohlrohr in die Handhabe hineinreichen. Hierdurch wird es auf einfache Weise ermöglicht, dass eine dem ersten Übertragungselement zugeordnete Betätigungseinrichtung, die innerhalb oder an der Handhabe angeordnet ist, an dem ersten und dem zweiten Hohlrohr angreift und dadurch das erste Funktionselement der Werkzeuganordnung über das durch das erste und das zweite Hohlrohr sowie das erste und das zweite Halbrohr gebildete erste Übertragungselement mittels der Betätigungseinrichtung betätigt werden kann.

Weiterhin ist es bevorzugt, dass das zweite Übertragungselement durch das Schaftgehäuse hindurch verläuft und proximalseitig in die Handhabe hineinreicht. Hierfür ist das zweite Übertragungselement insbesondere derart ausgebildet, dass es proximalseitig über das Schaftgehäuse oder, sofern zwischen dem Schaftgehäuse und der Handhabe ein Verbindungsrohr vorgesehen ist, über das proximale Ende des Verbindungsrohrs hinausragt. Hierdurch wird es ermöglicht, dass eine dem zweiten Übertragungselement zugeordnete Betätigungseinrichtung, die in oder an der Handhabe angeordnet ist, an dem zweiten Übertragungselement angreift, so dass das zweite Funktionselement der Werkzeuganordnung über das zweite Übertragungselement mittels der Betätigungseinrichtung betätigt werden kann.

In vorteilhafter Weise kann eine weitere Spülöffnung in dem ersten Übertragungselement vorgesehen sein, insbesondere in dem ersten Hohlrohr. Die weitere Spülöffnung kann durch eine Verschiebung des ersten Hohlrohrs in Längsrichtung des Schafts in Überlappung mit der Spülöffnung des Außenrohrs bzw. des Schaftgehäuses bringbar sein. Hierdurch wird die Einleitung einer Reinigungsflüssigkeit oder eines Sterilisationsfluids in einen Zwischenraum zwischen dem ersten und dem zweiten Hohlrohr ermöglicht. Alternativ kann ein Spalt zwischen dem ersten und dem zweiten Halbrohr zur Einleitung von Spülfluid in den Innenraum des ersten Übertragungselements nutzbar sein. Hierdurch wird das medizinische Instrument hinsichtlich der Möglichkeit zur Reinigung und/oder Sterilisation weiter verbessert.

Gemäß einer bevorzugten Ausführungsform der Erfindung ist zwischen einer Innenfläche des zweiten Hohlrohrs und einer Außenfläche des zweiten Übertragungselements eine ebenfalls im Wesentlichen ringförmige dritte Dichtung angeordnet. Sofern in dem ersten Übertragungselement eine Spülöffnung vorgesehen ist, ist die dritte Dichtung proximalseitig dieser Spülöffnung angeordnet. Hierdurch wird es ermöglicht, eine Reinigungsflüssigkeit oder ein Sterilisationsfluid in einen Zwischenraum zwischen dem ersten und dem zweiten Übertragungselement einzuleiten, ohne dass dieses in die Handhabe gelangen kann; ebenso kann ein Eindringen von Körperflüssigkeiten bzw. Spül- oder Insufflationsfluid in die Handhabe sicherer vermieden werden.

Gemäß einer bevorzugten Ausführungsform der Erfindung ist das zweite Übertragungselement als Zugstange ausgebildet. Die Zugstange ist relativ zum Außenrohr und relativ zum ersten Übertragungselement in Längsrichtung des Schafts verschiebbar. Insbesondere kann die Zugstange mit einer entsprechenden Betätigungseinrichtung der Handhabe verschiebbar sein. Die Zugstange kann Zugkräfte und zumindest in begrenztem Maße auch Schubkräfte übertragen. Diese Ausführungsform der Erfindung ist besonders einfach und robust ausgebildet und ermöglicht die Übertragung hoher Kräfte zur Betätigung des zweiten Funktionselements.

Gemäß einer weiteren bevorzugten Ausführungsform der Erfindung ist das zweite Übertragungselement als im Wesentlichen hohlzylindrisches Innenrohr ausgebildet, wobei innerhalb des Innenrohrs ein langerstrecktes drittes Übertragungselement verläuft. Das Innenrohr kann zur Betätigung des zweiten Funktionselements relativ zum Außenrohr verschiebbar und/oder um seine Längsachse drehbar sein. Hierfür kann dem Innenrohr eine entsprechende Betätigungseinrichtung der Handhabe zugeordnet sein. Das dritte Übertragungselement kann beispielsweise eine oder mehrere elektrische Leitungen zur Übertragung einer Hochfrequenzspannung von der Handhabe zur Werkzeuganordnung umfassen; hierfür kann an die Handhabe eine Hochfrequenzspannungsquelle anschließbar sein. Dadurch, dass das zweite Übertragungselement als Innenrohr ausgebildet ist, innerhalb dessen ein drittes Übertragungselement angeordnet ist, sind weitere Betätigungsmöglichkeiten der Werkzeuganordnung realisierbar, wodurch die Anwendungsmöglichkeiten des medizinischen Instruments erweitert werden können.

Vorzugsweise ist das erste Funktionselement der Werkzeuganordnung als gegen eine Längsachse des Schafts abwinkelbarer Biegeabschnitt ausgebildet, wobei ein proximales Ende des Biegeabschnitts an einem distalen Ende des Schafts angeordnet ist und an einem distalen Ende des Biegeabschnitts das zweite Funktionselement der Werkzeuganordnung angeordnet ist. Der Biegeabschnitt ist durch eine Verschiebung des ersten Halbrohrs gegenüber dem zweiten Halbrohr und somit durch eine Verschiebung des ersten Hohlrohrs relativ zum zweiten Hohlrohr gegenüber der Längsachse des Schafts abwinkelbar. Hierdurch wird es ermöglicht, dass der Biegeabschnitt von einer mit dem ersten Übertragungselement verbundenen Betätigungseinrichtung der Handhabe ansteuerbar ist. Der Biegeabschnitt weist insbesondere einen näherungsweise gleichen Außendurchmesser auf wie ein distaler Abschnitt des Außenrohrs des Schafts, so dass der Biegeabschnitt in einer nicht abgewinkelten Stellung eine gerade Fortsetzung des Schafts in distaler Richtung bildet. Hierdurch wird bei einem endoskopischen Eingriff die Einführung des erfindungsgemäßen medizinischen Instruments in einen Hohlraum, beispielsweise durch eine endoskopische Einführvorrichtung, erleichtert.

Gemäß einer bevorzugten Ausführungsform der Erfindung ist das zweite Funktionselement als Arbeitswerkzeug ausgebildet, das mindestens ein bewegliches Werkzeugelement umfasst, das über das zweite Übertragungselement mittels einer dem zweiten Übertragungselement zugeordneten Betätigungseinrichtung der Handhabe betätigbar ist. Das Arbeitswerkzeug kann insbesondere zwei Werkzeugelemente umfassen, die relativ zueinander schwenkbar sind, so dass diese zum Öffnen und Schließen des Arbeitswerkzeugs zusammenwirken können. Dabei können beide Werkzeugelemente relativ zu einer Basis des zweiten Funktionselements, die insbesondere an dem distalen Ende des ersten Funktionselements angeordnet ist, schwenkbar sein oder es kann ein Werkzeugelement an der Basis feststehend angeordnet und nur das andere schwenkbar ausgebildet sein. Die Werkzeugelemente können beispielsweise als Branchen einer Schere oder als Maulteile einer Fasszange ausgebildet sein. Insbesondere kann es vorgesehen sein, dass das mindestens eine bewegliche Werkzeugelement mittels der Betätigungseinrichtung, die proximalseitig an dem zweiten Übertragungselement angreift kann, bewegt werden kann, beispielsweise eine Schere oder Zange geöffnet oder geschlossen werden kann. Das mindestens eine bewegliche Werkzeugelement ist vorzugsweise durch eine Verschiebung des zweiten Übertragungselements betätigbar. Hierdurch wird ein einfach zu betätigendes und für eine Vielzahl von Manipulationen verwendbares Arbeitswerkzeug geschaffen. Vorzugsweise ist das Arbeitswerkzeug derart ausgebildet, dass es in einer Ausgangsstellung des mindestens einen beweglichen Werkzeugelements und bei einer gestreckten Stellung des Biegeabschnitts innerhalb einer distalseitigen Verlängerung der Außenfläche des Außenrohrs angeordnet ist; hierdurch wird die Einführung des erfindungsgemäßen medizinischen Instruments in einen körperinneren Hohlraum erleichtert.

Weiterhin ist es bevorzugt, dass das Arbeitswerkzeug um eine Längsachse des Arbeitswerkzeugs drehbar ist. Vorzugsweise ist das zweite Übertragungselement als Innenrohr ausgebildet, wobei eine Rotation des Innenrohrs um die Längsachse des Schafts eine entsprechende Drehung des Arbeitswerkzeugs, insbesondere einer am distalen Ende des Biegeabschnitts angeordneten Basis des Arbeitswerkzeugs, um seine Längsachse bewirkt. Hierfür kann das Innenrohr durch den Biegeabschnitt der Werkzeuganordnung hindurchgeführt und entsprechend biegsam ausgebildet sein. Auf diese Weise wird eine besonders vielseitige Verwendbarkeit des Arbeitswerkzeugs ermöglicht.

Gemäß einer weiteren Ausführungsform der Erfindung weist das medizinische Instrument die Merkmale des Oberbegriffs von Anspruch 1 auf sowie, wie oben beschrieben, in einem proximalen Endabschnitt des Schafts ein gegenüber dem Schaft erweitertes Schaftgehäuse, das mit dem Außenrohr verbunden ist. Bei dieser Ausführungsform erstrecken sich das erste Halbrohr und das zweite Halbrohr des ersten Übertragungselements durch das Schaftgehäuse hindurch, wobei zwischen einer Innenfläche des Schaftgehäuses und den Außenflächen des ersten und des zweiten Halbrohrs eine Dichtung angeordnet ist, die vorzugsweise im Wesentlichen ringförmig ausgebildet ist und die im Folgenden als äußere Dichtung bezeichnet wird. Die äußere Dichtung liegt dichtend an einer vorzugsweise zylindrischen Innenfläche des Schaftgehäuses und ebenfalls dichtend an der Außenfläche des ersten Übertragungselements, das durch das erste und das zweite Halbrohr gebildet wird, an. Die äußere Dichtung kann insbesondere mit einer Innenfläche des Schaftgehäuses fest verbunden sein und beispielsweise in eine umlaufende Nut in der Innenfläche des Schaftgehäuses eingesetzt sein. Im Übrigen kann das medizinische Instrument gemäß dieser Ausführungsform wie zuvor beschrieben ausgebildet sein. Dadurch, dass zwischen der Innenfläche des Schaftgehäuses und der Außenfläche des ersten Übertragungselements eine Dichtung angeordnet ist, kann auch bei dieser Ausführungsform der Erfindung ein Eintreten von Reinigungsflüssigkeit oder Sterilisationsfluid in die Handhabe verhindert werden.

Vorzugsweise sind das erste Halbrohr und das zweite Halbrohr mit ihren offenen Längsseiten einander zugewandt und bilden zwischen ihren Längsschnittflächen jeweils einen Spalt; hierdurch kann erreicht werden, dass das erste gegenüber dem zweiten Halbrohr besonders reibungsarm verschiebbar ist. Weiterhin vorzugsweise ist zwischen einer Innenfläche des ersten Übertragungselements, d. h. zwischen den Innenflächen des ersten und des zweiten Halbrohrs, und der Außenfläche des zweiten Übertragungselements eine weitere Dichtung angeordnet, die ebenfalls im Wesentlichen ringförmig ist und die im Folgenden als innere Dichtung bezeichnet wird. Die zwischen dem Schaftgehäuse und dem ersten und dem zweiten Halbrohr angeordnete äußere Dichtung weist in diesem Fall zwei einander gegenüberliegend nach innen ragende, sich in Längsrichtung des Schafts erstreckende Stege auf, die jeweils in einen der Spalte hineinragen und dichtend an den Längsschnittflächen der Halbrohre sowie an der inneren Dichtung anliegen. Hierdurch kann die Abdichtung weiter verbessert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung zweier bevorzugten Ausführungsbeispiele und der beigefügten Zeichnung. Es zeigen:
Fig. 1a und 1b einen Abschnitt des Schafts eines medizinischen Instruments gemäß einem ersten Ausführungsbeispiel der Erfindung in einer Schrägansicht sowie in einer in Längsrichtung geschnittenen Ansicht, jeweils in einer ersten Relativposition des ersten und des zweiten Halbrohrs;
Fig. 2a und 2b den Abschnitt des Schafts gemäß Fig. 1a und 1b, jedoch in einer zweiten Relativposition des ersten und des zweiten Halbrohrs;
Fig. 3a und 3b den Abschnitt des Schafts gemäß Fig. 1a und 1b, jedoch in einer dritten Relativposition des ersten und des zweiten Halbrohrs;
Fig. 4a und 4b einen Abschnitt des Schafts eines medizinischen Instruments gemäß einem zweiten Ausführungsbeispiel der Erfindung in einer Schrägansicht sowie in einer Ausschnittsvergrößerung;
Fig. 5a und 5b eine äußere Dichtung gemäß dem zweiten Ausführungsbeispiel der Erfindung in einer Schräg- und einer stirnseitigen Ansicht;
Fig. 6 ein medizinisches Instrument gemäß dem ersten und dem zweiten Ausführungsbeispiel der Erfindung in einer Gesamtansicht.

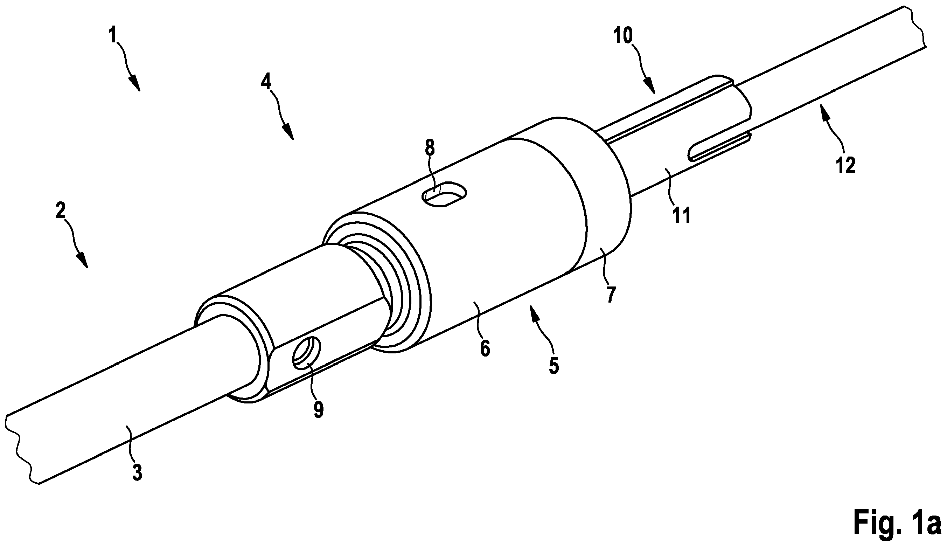

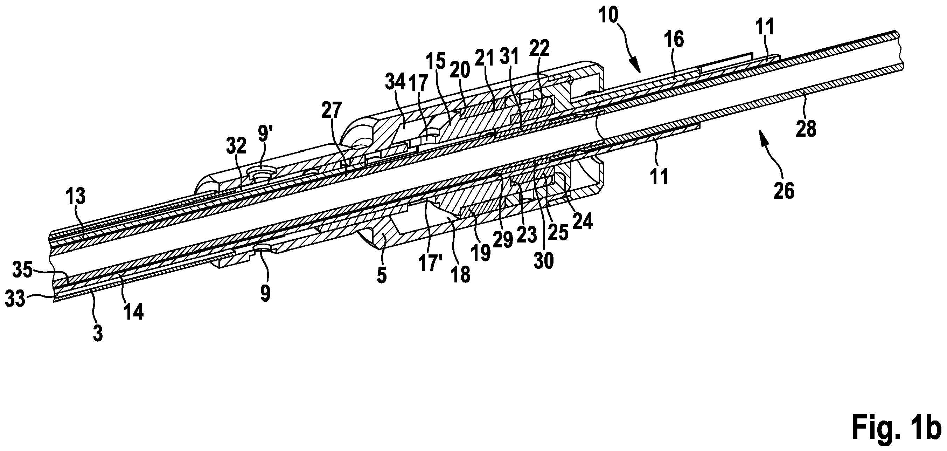

In Fig. 1a und 1b ist ein Abschnitt des Schafts 1 eines medizinischen Instruments gemäß einem ersten Ausführungsbeispiel der Erfindung in einer Schrägansicht sowie in einer Schnittansicht dargestellt. Das dargestellte medizinische Instrument ist als endoskopisches Instrument ausgebildet. Wie in Fig. 1a gezeigt ist, umfasst der Schaft 1 einen distalen Abschnitt 2 mit einem hohlzylindrischen Außenrohr 3, wobei der distale Abschnitt 2 zur Einführung in einen körperinneren Hohlraum eines menschlichen oder tierischen Körpers bestimmt ist, sowie einen proximalen Endabschnitt 4 mit einem gegenüber dem Außenrohr 3 erweiterten Schaftgehäuse 5, das bei einem endoskopischen Eingriff außerhalb des Körpers verbleibt. Das Schaftgehäuse 5 umfasst einen Hauptkörper 6 und eine an diesen proximalseitig angesetzte Endkappe 7 sowie gegebenenfalls weitere, in Fig. 1a und 1b nicht gezeigte Bauelemente, wie etwa einen Spülanschlussstutzen. Der Hauptkörper 6 weist eine erste Spülöffnung 8 sowie eine zweite Spülöffnung 9 auf, durch die eine Reinigungsflüssigkeit ein- oder ausleitbar ist. Bei dem dargestellten Ausführungsbeispiel ist das Schaftgehäuse 5 außenseitig abschnittsweise im Wesentlichen zylindrisch, jedoch mit einem größeren Außendurchmesser als das Außenrohr 3 ausgebildet. Wie in Fig. 1a angedeutet ist, ragen proximale Abschnitte eines ersten Hohlrohrs 10, eines zweiten Hohlrohrs 11 und eines innerhalb des zweiten Hohlrohrs 11 verlaufenden Innenrohrs 12 proximalseitig aus dem Schaftgehäuse 5 heraus und in eine in Fig. 1a und 1b nicht dargestellte Handhabe, die proximalseitig an das Schaftgehäuse 5 anschließt, hinein. Wie in Fig. 1a und 1b ebenfalls nicht dargestellt ist, ist an einem distalen Ende des distalen Abschnitts 2 des Schafts 1 eine Werkzeuganordnung angeordnet, mit der in einem körperinneren Hohlraum chirurgische Manipulationen ausgeführt werden können.

In Fig. 1b ist der proximale Endabschnitt 4 in einem Längsschnitt gezeigt. Die Schnittebene ist eine in der Darstellung der Figur 1a horizontale Ebene, in der die Mittellängsachse des Schafts 1 verläuft, die der Mittellängsachse des Außenrohrs 3 entspricht. In der in Fig. 1b gezeigten Schnittansicht sind die im distalen Teil des Schaftgehäuses 5 vorgesehene Spülöffnung 9 sowie die dieser gegenüberliegende Spülöffnung 9' angeschnitten. Die Spülöffnung 8 ist in Fig. 1b nicht zu erkennen. Das Außenrohr 3 ist in den distalen Teil des Hauptkörpers 6 abgedichtet eingesetzt und fest mit diesem verbunden, beispielsweise durch Kleben, Löten oder Schweißen. Der in den Hauptkörper 6 eingesetzte Abschnitt des Außenrohrs 3 ist ausgeschnitten, um die Spülöffnungen 9, 9' nicht zu verdecken. Innerhalb des Außenrohrs 3 sind ein erstes Halbrohr 13 und ein zweites Halbrohr 14 angeordnet, die jeweils einen halbkreisförmigen Querschnitt aufweisen und mit ihren offenen Seiten aneinander angesetzt sind. Die Halbrohre 13, 14, die in dem in Fig. 1b dargestellten Längsschnitt jeweils etwa in einer Mittellängsebene geschnitten sind, bilden gemeinsam somit eine im Wesentlichen hohlzylindrische Rohranordnung. Das erste Halbrohr 13 und das zweite Halbrohr 14 sind jeweils in Längsrichtung des Außenrohrs 3 in diesem verschiebbar gelagert. Das erste Halbrohr 13 ist in seinem proximalen Endabschnitt fest mit dem ersten Hohlrohr 10 verbunden, beispielsweise durch Kleben, Löten oder Schweißen. Das erste Hohlrohr 10 besteht aus einem distalen Rohrabschnitt 15 und einem proximalen Rohrabschnitt 16, wobei der distale Rohrabschnitt 15 distalseitig mit dem ersten Halbrohr 13 und proximalseitig mit dem proximalen Rohrabschnitt 16 verbunden ist. Das erste Hohlrohr 10 ist gemeinsam mit dem ersten Halbrohr 13 in Längsrichtung des Schafts 1 verschiebbar. Der distale Rohrabschnitt 15 weist Spülöffnungen 17, 17' sowie ggf. weitere Öffnungen auf und ist im Übrigen als umfangsmäßig geschlossenes Rohr ausgebildet, d. h. als Rohr, das sich von seiner Mittellängsachse aus gesehen über 360 ° erstreckt. Der distale Rohrabschnitt 15 ist abschnittsweise ebenfalls als umfangsmäßig geschlossenes Rohr und in einem proximalen Endabschnitt als Halbrohr ausgebildet. Das zweite Halbrohr 14 ist einstückig mit dem zweiten Hohlrohr 11 verbunden, wobei das zweite Hohlrohr 11 abschnittsweise umfangsmäßig geschlossen ist.

Der Hauptkörper 6 des Schaftgehäuses 5 weist in seinem proximalen Teil eine zylindrische Innenfläche 18 auf. An der Innenfläche 18 liegt eine ringförmig ausgebildete erste Dichtung 19 dichtend an, die auf der Außenseite des ersten Hohlrohrs 10 in eine zwischen einer Stufe 20 und dem distalen Ende des proximalen Rohrabschnitts 16 gebildete ringförmige Ausnehmung 21 eingesetzt ist. Auf der Innenseite des ersten Hohlrohrs 10 ist in eine ringförmige Ausnehmung 22, die zwischen einer Stufe 23 des distalen Rohrabschnitts 15 und einer Stufe 24 des proximalen Rohrabschnitts 16 gebildet ist, eine zweite, ebenfalls ringförmig ausgebildete Dichtung 25 eingesetzt, die auf einer zylindrischen Außenfläche eines umfangsmäßigen geschlossenen Abschnitts des zweiten Hohlrohrs 11 dichtend anliegt. Die Dichtungen 19, 25 sind beispielsweise als kurze, zylindrische Schlauchabschnitte ausgeführt.

Innerhalb der durch das erste Halbrohr 13 und das zweite Halbrohr 14 gebildeten Rohranordnung sowie innerhalb des zweiten Hohlrohrs 11 ist ein Innenrohr 26 angeordnet, das aus einem distalen Rohrabschnitt 27 und einem proximalen Rohrabschnitt 28, die fest miteinander verbunden sind, zusammengesetzt ist. Das Innenrohr 26 weist eine im Wesentlichen zylindrisch ausgebildete Innenfläche auf, in der weitere, in den Figuren nicht dargestellte Übertragungselemente verlaufen können. Zwischen einer Stufe 29 des distalen Rohrabschnitts 27 und dem distalen Ende des proximalen Rohrabschnitts 28 ist eine ringförmige Ausnehmung 30 gebildet, in die eine dritte Dichtung 31 eingesetzt ist, die ebenfalls ringförmig ausgebildet ist, beispielsweise als kurzer zylindrischer Schlauchabschnitt. Die dritte Dichtung 31 liegt dichtend an einer zylindrischen Innenfläche des zweiten Hohlrohrs 11 an. Der proximale Rohrabschnitt 16 des ersten Hohlrohrs 10, das zweite Hohlrohr 11 und das Innenrohr 26 ragen proximalseitig über das Schaftgehäuse 5 hinaus und in eine proximalseitig an das Schaftgehäuse 5 anschließende, nicht dargestellte Handhabe hinein, wo sie mit entsprechenden Betätigungseinrichtungen verbunden sind.

Die Spülöffnungen 9, 9' stehen in Verbindung mit einem ersten Hohlraum 32 innerhalb des Schaftgehäuses 5, der mit einem Zwischenraum 33 zwischen dem Außenrohr und dem ersten Halbrohr 13 bzw. dem zweiten Halbrohr 14 in Fluidverbindung steht. Die in Fig. 1b nicht sichtbare Spülöffnung 8 mündet in einen zweiten Hohlraum 34 des Schaftgehäuses 5, der über die Spülöffnungen 17, 17' mit einem Zwischenraum 35 zwischen dem ersten Halbrohr 13 bzw. dem zweiten Halbrohr 14 und dem Innenrohr 26 in Verbindung steht. Durch die Spülöffnungen 8, 17, 17' kann eine Reinigungsflüssigkeit oder ein Sterilisationsfluid in den Schaft 1 eingeleitet werden und durch den Zwischenraum 35 zwischen den Halbrohren 13, 14 und dem Innenrohr 26 in distaler Richtung geleitet werden und dort austreten, ebenso wie durch die Spülöffnungen 9, 9' und durch den Zwischenraum 33 zwischen dem Außenrohr und den Halbrohren 13, 14. Die Zwischenräume 33, 35 ermöglichen auch ein ausreichendes Spiel, so dass eine reibungsarme Verschiebung der Halbrohre 13, 14 und des Innenrohrs 26 in axialer Richtung möglich ist.

Die beschriebene Durchleitung von Reinigungsflüssigkeit ermöglicht eine Reinigung der in dem Außenschaft 3 angeordneten Bauelemente sowie ggf. weiterer Bauelemente der in den Figuren nicht dargestellten Werkzeuganordnung; ebenso kann durch Durchleitung eines Sterilisationsfluids eine Sterilisation ermöglicht werden. Gleichzeitig wird durch die Dichtungen 19, 25, 31 ein Austreten der Reinigungsflüssigkeit bzw. des Sterilisationsfluids in proximaler Richtung und damit ein Eindringen in die proximalseitig an das Schaftgehäuse 5 angesetzte Handhabe, in die das erste Hohlrohr 10, das zweite Hohlrohr 11 und das Innenrohr 26 hineinragen, vermieden. Die Dichtungen 19, 25, 31 können aus PTFE (Teflon) oder einem anderen geeigneten Dichtungsmaterial bestehen.

Zur Betätigung eines ersten Funktionselements der am distalen Ende des distalen Abschnitts 2 des Schafts 1 angeordneten Werkzeuganordnung, beispielsweise zur Abwinkelung eines Biegeabschnitts der Werkzeuganordnung gegenüber der Längsachse des Schafts 1 des medizinischen Instruments, werden das erste Halbrohr 13 und das zweite Halbrohr 14 gegeneinander verschoben. Hierfür ist innerhalb der Handhabe eine Betätigungseinrichtung vorgesehen, die mit dem ersten Hohlrohr 10 und dem zweiten Hohlrohr 11 verbunden ist und die eine gegensinnige gleich große Verschiebung des ersten Hohlrohrs 10 und des zweiten Hohlrohrs 11 gegenüber der Handhabe und dem mit dieser verbundenen Schaftgehäuse 5 bewirkt. Diese Betätigungseinrichtung kann etwa ein motorisch betreibbares Zahnrad umfassen, das zwischen einer mit dem ersten Hohlrohr 10 und einer mit dem zweiten Hohlrohr 11 verbundenen Zahnstange angeordnet ist und in diese eingreift. Die gegensinnige Verschiebung des ersten Hohlrohrs 10 und des zweiten Hohlrohrs 11 bewirkt eine Verschiebung des ersten Halbrohrs 13 gegenüber dem zweiten Halbrohr 14 und eine entsprechende Betätigung des ersten Funktionselements, beispielsweise über Schiebeelemente eine Abwinkelung eines Biegeabschnitts der Werkzeuganordnung.

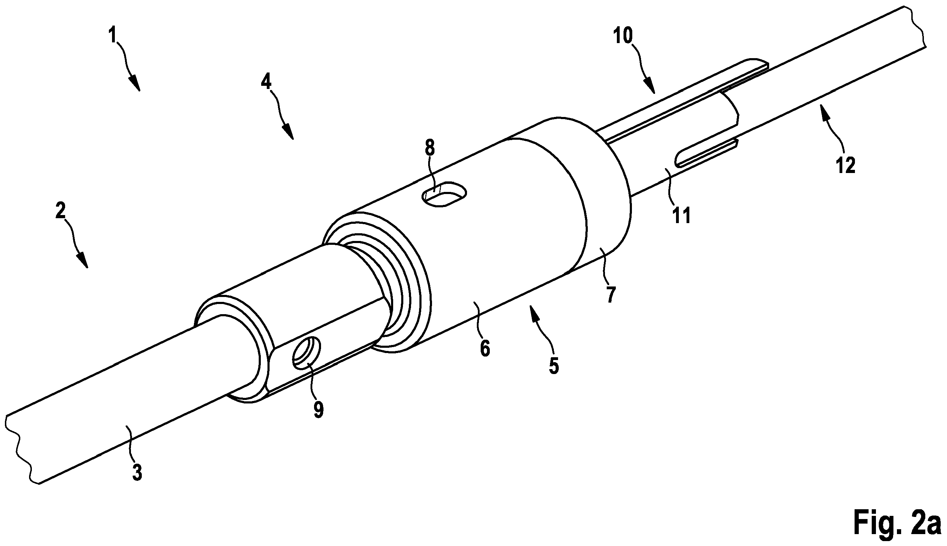

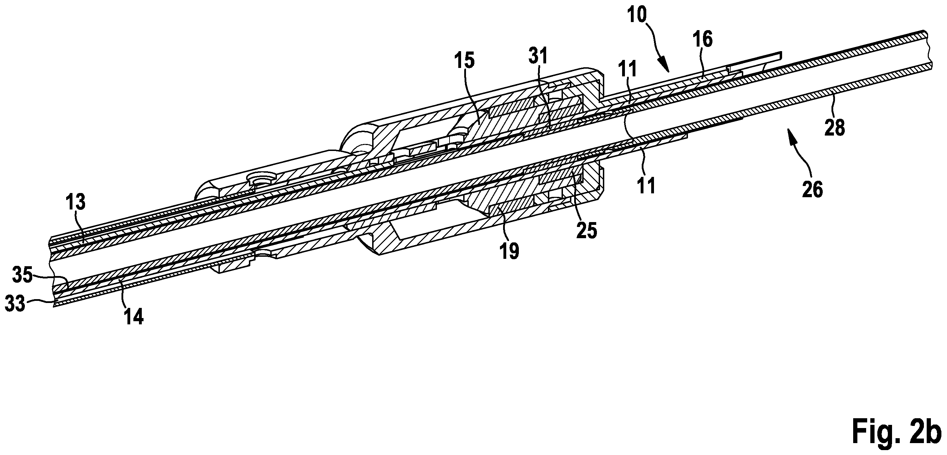

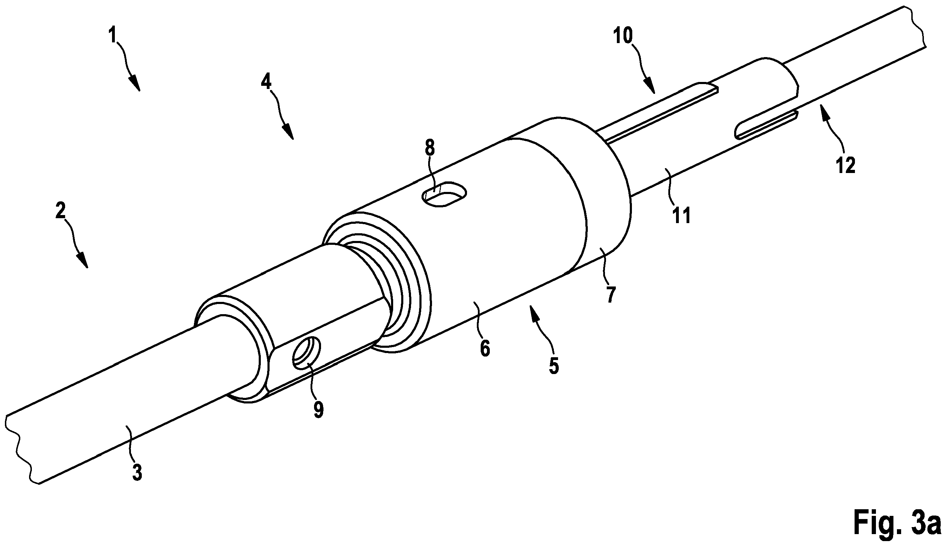

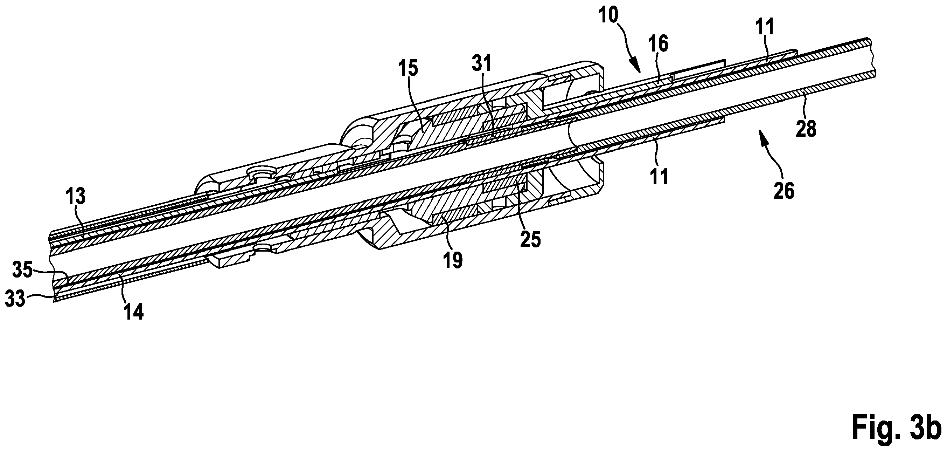

In den Figuren 2a bis 3b sind in Darstellungen, die jeweils den in Fig. 1a und Fig.1b gezeigten Ansichten entsprechen, die beiden Extrempositionen der Relativbewegung des ersten Hohlrohrs 10 relativ zum zweiten Hohlrohr 11 bzw. des ersten Halbrohrs 13 relativ zum zweiten Halbrohr 14 dargestellt, wohingegen die Figuren 1a und 1b jeweils eine mittlere Position zeigen. In der in Fig. 2a und 2b gezeigten Position ist das erste Hohlrohr 10 gegenüber der in den Figuren 1a und 1b gezeigten Stellung in proximaler Richtung und das zweite Hohlrohr 11 in distaler Richtung verschoben. Dementsprechend liegt die erste Dichtung 19 an einem nach proximal verschobenen Bereich der Innenfläche 18 des Hauptkörpers 6 des Schaftgehäuses 5 an; die zweite Dichtung 25 und, wenn die Position des Innenrohrs 26 unverändert ist, die dritte Dichtung 31 sind auf der Außen- bzw. der Innenfläche des zweiten Hohlrohrs 11 gegenüber der in Fig. 1a und 1b gezeigten Stellung in proximaler Richtung verschoben. Andererseits ist in Fig. 3a und 3b eine maximale Verschiebung des ersten Hohlrohrs 10 gegenüber dem zweiten Hohlrohr 11 in distaler Richtung dargestellt. Hierbei liegt die erste Dichtung 19 an einem in distaler Richtung verschobenen Bereich der Innenfläche 18 des Hauptkörpers 6 an und die zweite Dichtung 25 und die dritte Dichtung 31 sind auf der Außen- bzw. der Innenfläche des zweiten Hohlrohrs 11 in distaler Richtung verschoben. Wie in den Figuren 1b und 2b zu erkennen ist, besteht auch in den beiden Endstellungen die oben beschriebene Fluidverbindung zwischen der Spülöffnung 8 und dem Zwischenraum 35 sowie zwischen den Spülöffnungen 9, 9' und dem Zwischenraum 33. Die Einleitung einer Reinigungsflüssigkeit oder eines Sterilisationsfluids ist daher unabhängig von der Betätigungsstellung des ersten und ebenso des zweiten Funktionselements möglich.

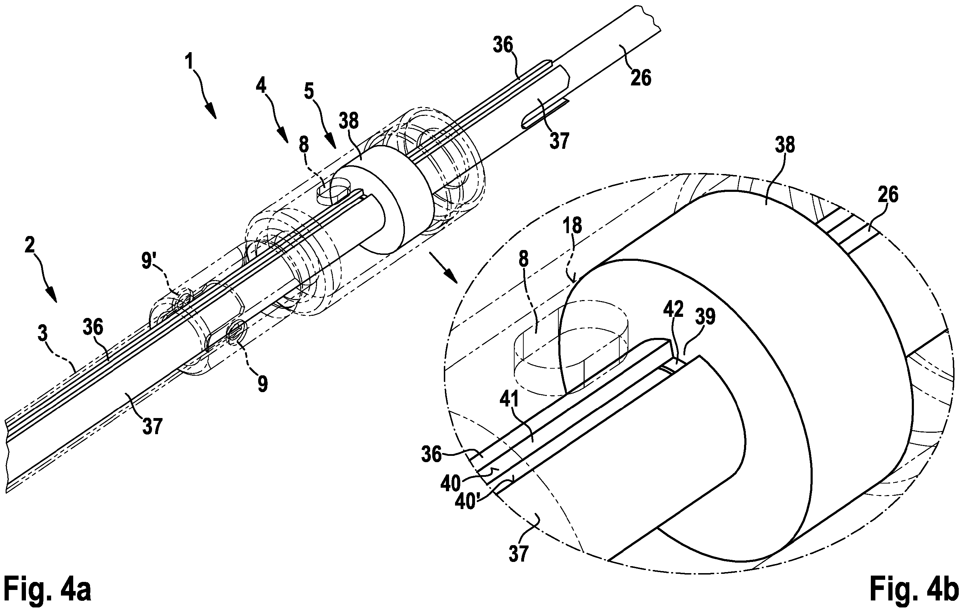

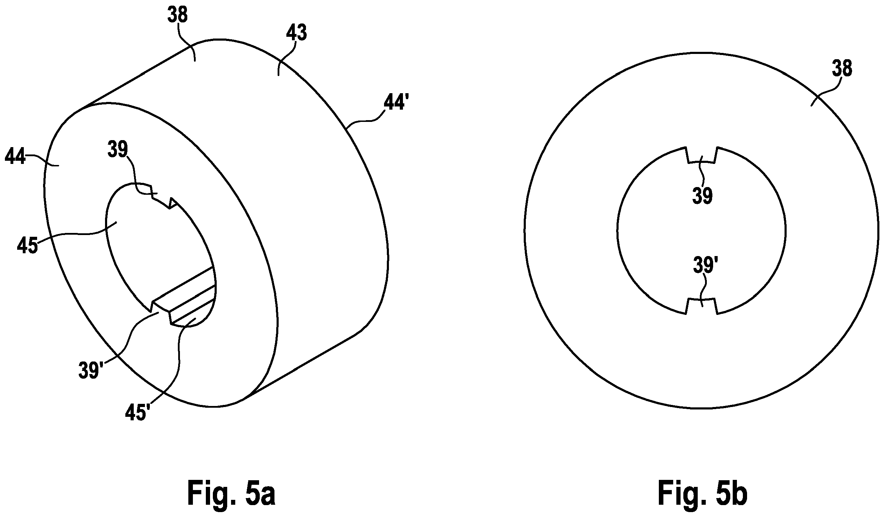

In Fig. 4a ist ein Abschnitt des Schafts 1 eines medizinischen Instruments gemäß einem zweiten Ausführungsbeispiel der Erfindung in einer der Fig. 1a entsprechenden Ansicht gezeigt, wobei das Schaftgehäuse 5 transparent dargestellt ist. Fig. 4b zeigt eine Ausschnittsvergrößerung. Wie in Fig. 4a gezeigt ist, verlaufen ein erstes Halbrohr 36 und ein zweites Halbrohr 37 sowie das Innenrohr 26 durch das Schaftgehäuse hindurch und ragen proximalseitig aus diesem hinaus, so dass sie in eine an das Schaftgehäuse 5 angesetzte, nicht dargestellte Handhabe hinein reichen, wo entsprechende Betätigungseinrichtungen vorgesehen sind. Zwischen der zylindrischen Innenfläche 18 des Schaftgehäuses 5 und den Halbrohren 36, 37 ist eine im Wesentlichen ringförmige Dichtung 38 angeordnet, die außerhalb der Halbrohre 36, 37 abdichtet und hier auch als äußere Dichtung 38 bezeichnet wird. Die äußere Dichtung 38 ist außenseitig zylindrisch geformt und liegt dichtend an der Innenfläche 18 des Schaftgehäuses 5 an. Auf ihrer Innenseite liegt die äußere Dichtung 38 dichtend an den Außenseiten der Halbrohre 36, 37 an und greift mit einem nach innen ragenden Vorsprung, der als in axialer Richtung verlaufender Steg 39 ausgebildet ist (s. Fig. 5a, 5b), in einen zwischen den Längsschnittflächen 40, 40' der Halbrohre 36, 37 gebildeten Spalt 41 hinein. Auf der dem Steg 39 gegenüberliegenden Seite der äußeren Dichtung 38 ragt ebenfalls ein axialer Steg 39' nach innen, der in einen entsprechenden Spalt zwischen den anderen, in Fig. 4a und 4b nicht sichtbaren Längsschnittflächen der Halbrohre 36, 37 eingreift. Das Innenrohr 26 trägt in einer ringförmigen Ausnehmung eine weitere Dichtung 42, die innerhalb der Halbrohre 36, 37 abdichtet und die daher hier als innere Dichtung bezeichnet wird. Die innere Dichtung 42 ist im Wesentlichen wie die bei dem ersten Ausführungsbeispiel beschriebene dritte Dichtung 31 ausgebildet, dichtet jedoch zwischen dem Innenrohr 26 und den Halbrohren 36, 37 sowie den Stegen 39, 39' der äußeren Dichtung 38 ab. Die äußere Dichtung 38 kann beispielsweise an der Innenfläche 18 des Schaftgehäuses 5 befestigt sein und bei einer axialen Verschiebung der Halbrohre 36, 37 sowie des Innenrohrs 26 auf den Halbrohren 36, 37 sowie auf der inneren Dichtung 42 dichtend gleiten.

In Fig. 5a und 5b ist die äußere Dichtung 38 näher dargestellt. Die äußere Dichtung 38 weist eine zylindrische Außenfläche 43 auf und in axialer Richtung im Wesentlichen ebene Endflächen 44, 44'. Die Innenfläche der äußeren Dichtung 38 weist zwei näherungsweise halbzylindrische Bereiche 45, 45' auf, die durch die beiden nach innen vorspringenden, in axialer Richtung verlaufenden Stege 39, 39` voneinander getrennt sind. Die äußere Dichtung 38 kann beispielsweise aus Silikon oder einem anderen geeigneten Dichtungsmaterial bestehen.

Im Übrigen kann das medizinische Instrument gemäß dem ersten und dem zweiten Ausführungsbeispiel der Erfindung wie in der Europäischen Patentanmeldung EP 2 837 354 A1 beschrieben ausgebildet sein. Insbesondere kann die Werkzeuganordnung, die an dem distalen Ende des Schafts 2 angeordnet ist und die in den Figuren 1a bis 5b nicht dargestellt ist, wie in EP 2 837 354 A1 beschrieben ausgebildet sein und einen Biegeabschnitt und einen Greifer umfassen. Ebenso kann die in den Figuren 1a bis 5b nicht dargestellte Handhabe gemäß EP 2 837 354 A1 ausgebildet sein.

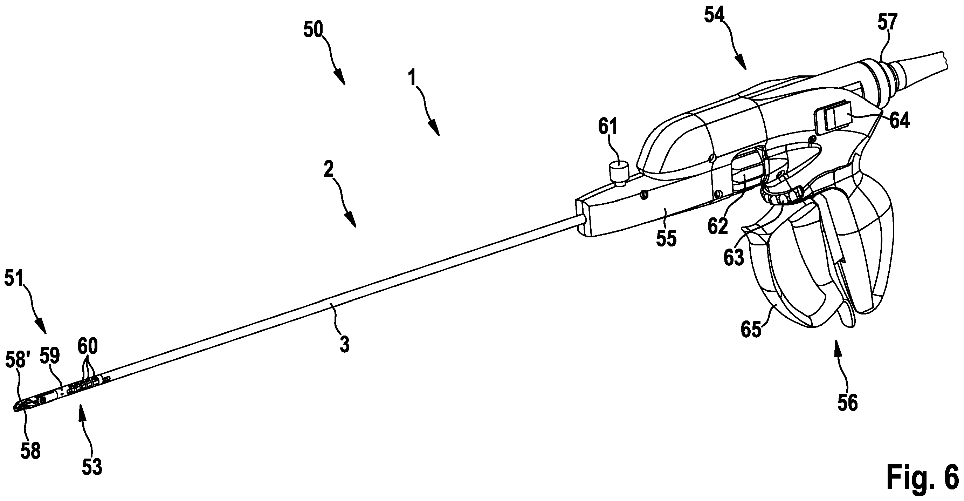

Ein medizinisches Instrument, dessen Schaft wie zuvor gemäß dem ersten und dem zweiten Ausführungsbeispiel beschrieben ausgebildet ist, kann insgesamt wie in Fig. 6 gezeigt ausgestaltet sein. Das medizinische Instrument 50, das als endoskopisches Instrument ausgebildet ist, weist einen Schaft 1 mit einem distalen Abschnitt 2, eine Werkzeuganordnung 51 mit einem als Zange 52 ausgebildeten Arbeitswerkzeug und einem Biegeabschnitt 53 sowie eine Handhabe 54 mit einem Gehäuse 55, einem Handgriff 56 und einem Anschluss 57 auf. Ein proximaler Endabschnitt des Schafts 1 ist in dem Gehäuse 55 aufgenommen. Der distale Abschnitt 2 und der proximale Endabschnitt sind gemäß dem zuvor beschriebenen ersten oder zweiten Ausführungsbeispiel ausgebildet.

Die Zange weist zwei Maulteile 58, 58' auf, die schwenkbar an einer Basis 59 angelenkt sind. Die Basis 59 ist um ihre Längsachse, die in der in Fig. 6 gezeigten gestreckten Stellung des Biegeabschnitt 53 mit der Längsachse des distalen Abschnitts 2 des Schafts 1 zusammenfällt, drehbar. Der Biegeabschnitt 53 umfasst eine Mehrzahl von Gelenkelementen 60, die über Gelenke miteinander verbunden sind und mittels Schiebelementen gegeneinander geschwenkt werden können.

Das Gehäuse 55 der Handhabe 54 trägt einen Spülanschlussstutzen 61, an den ein Zu- oder Ableitungsschlauch für eine Reinigungsflüssigkeit anschließbar ist und der mit der Spülöffnung 8 oder 9 bzw. 9' (s. Fig. 1a bis 4b) verbunden ist. Am Gehäuse 55 sind Bedienelemente 62, 63, 64 zur manuellen oder motorischen Steuerung der Biegung des Biegeabschnitts 53, der Rotation der Basis 59 der Zange 52 um ihre Längsachse und zur Steuerung einer Beaufschlagung der Zange 52 mit monopolarer oder bipolarer Hochfrequenzspannung angeordnet. Dabei kann eine Kontaktierung zur Übertragung der Hochfrequenzspannung zur Zange 52, die um die Längsachse der Basis 59 drehbar ist, über Schleifkontakte erfolgen, insbesondere in dem Fall, dass das medizinische Instrument als Bipolarzange ausgebildet ist. Durch Bewegung des Griffrings 65 des Handgriffs 56 kann das Öffnen und Schließen der Zange 52 bewirkt werden.

Innerhalb des distalen Abschnitts 2 des Schafts 1 verlaufen ein erstes Übertragungselement zur Betätigung des Biegeabschnitts 53, ein zweites Übertragungselement zur Betätigung der Zange 52 und ein drittes Übertragungselement zur Übertragung der Hochfrequenzspannung zu den Maulteilen 58, 58'. Das erste und das zweite Übertragungselement verlaufen bis in die Handhabe 54 hinein, wo sie mit Betätigungseinrichtungen, die von den entsprechenden Bedienelementen 62, 63 und dem Griffring 65 gesteuert werden können, verbunden sind. Das dritte Übertragungselement ist mit dem Anschluss 57, an den eine Hochfrequenzspannungsquelle anschließbar ist, verbunden und kann durch das weitere Bedienelement 64 unterbrochen werden. Die genannten Übertragungselemente sowie der Schaft 1 sind wie zuvor beschrieben gemäß dem ersten oder dem zweiten Ausführungsbeispiel der Erfindung ausgebildet. Im Übrigen kann das medizinische Instrument 50 wie in der Europäischen Patentanmeldung EP 2 837 354 A1 beschrieben ausgebildet sein. Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Schaft
- 2: Distaler Abschnitt
- 3: Außenrohr
- 4: Proximaler Endabschnitt
- 5: Schaftgehäuse
- 6: Hauptkörper
- 7: Endkappe
- 8: Spülöffnung
- 9, 9': Spülöffnung
- 10: Hohlrohr
- 11: Hohlrohr
- 12: Innenrohr
- 13: Halbrohr
- 14: Halbrohr
- 15: Distaler Rohrabschnitt
- 16: Proximaler Rohrabschnitt
- 17, 17': Spülöffnung
- 18: Innenfläche
- 19: Dichtung
- 20: Stufe
- 21: Ausnehmung
- 22: Ausnehmung
- 23: Stufe
- 24: Stufe
- 25: Dichtung
- 26: Innenrohr
- 27: Distaler Rohrabschnitt
- 28: Proximaler Rohrabschnitt
- 29: Stufe
- 30: Ausnehmung
- 31: Dichtung
- 32: Hohlraum
- 33: Zwischenraum
- 34: Hohlraum
- 35: Zwischenraum
- 36: Halbrohr
- 37: Halbrohr
- 38: Dichtung
- 39, 39': Steg
- 40, 40': Längsschnittfläche
- 41: Spalt
- 42: Dichtung
- 43: Außenfläche
- 44, 44': Endfläche
- 45, 45': Bereich
- 50: Medizinisches Instrument
- 51: Werkzeuganordnung
- 52: Zange
- 53: Biegeabschnitt
- 54: Handhabe
- 55: Gehäuse
- 56: Handgriff
- 57: Anschluss
- 58, 58': Maulteil
- 59: Basis
- 60: Biegeelement
- 61: Spülanschlussstutzen
- 62: Bedienelement
- 63: Bedienelement
- 64: Bedienelement
- 65: Griffring

## Patentansprüche

1. Medizinisches Instrument (50) mit einem langerstreckten Schaft (1), einer an einem distalen Ende des Schafts (1) angeordneten Werkzeuganordnung (51) und einer an einem proximalen Ende des Schafts (1) angeordneten Handhabe (54) zur Betätigung der Werkzeuganordnung (51), wobei der Schaft (1) ein langerstrecktes Außenrohr (3) aufweist, innerhalb dessen ein erstes Übertragungselement zur Übertragung einer Betätigungsbewegung von der Handhabe (54) zur Werkzeuganordnung (51) zur Betätigung mindestens eines ersten Funktionselements der Werkzeuganordnung (51) angeordnet ist, wobei das erste Übertragungselement ein erstes Halbrohr (13, 36) und ein zweites Halbrohr (14, 37) umfasst, die zur Betätigung des mindestens einen ersten Funktionselements gegeneinander in einer Längsrichtung des Schafts (1) verschiebbar sind, und wobei in einem von dem ersten Halbrohr (13, 36) und dem zweiten Halbrohr (14, 37) gebildeten längs erstreckten Hohlraum ein zweites Übertragungselement zur Übertragung einer Betätigungsbewegung von der Handhabe (54) zur Werkzeuganordnung (51) zur Betätigung mindestens eines zweiten Funktionselements der Werkzeuganordnung (51) angeordnet ist, **dadurch gekennzeichnet, dass** in einem proximalen Endabschnitt (4) des Schafts (1) ein gegenüber dem Schaft (1) erweitertes Schaftgehäuse (5) angeordnet ist, das mit dem Außenrohr (3) verbunden ist, dass innerhalb des Schaftgehäuses (5) ein erstes Hohlrohr (10) angeordnet ist, das mit dem ersten Halbrohr (13) verbunden ist, und dass innerhalb des ersten Hohlrohrs (10) ein zweites Hohlrohr (11) angeordnet ist, das mit dem zweiten Halbrohr (14) verbunden ist, wobei zwischen einer Innenfläche (18) des Schaftgehäuses (5) und einer Außenfläche des ersten Hohlrohrs (10) eine ringförmige erste Dichtung (19) und zwischen einer Innenfläche des ersten Hohlrohrs (10) und einer Außenfläche des zweiten Hohlrohrs (11) eine ringförmige zweite Dichtung (25) angeordnet ist.

2. Medizinisches Instrument (50) nach Anspruch 1, **dadurch gekennzeichnet, dass** das Außenrohr (3) und/oder das Schaftgehäuse (5) eine Spülöffnung (8, 9, 9') aufweist und dass die erste Dichtung (19) proximalseitig der Spülöffnung (8, 9, 9') angeordnet ist.

3. Medizinisches Instrument (50) nach Anspruch 1 oder 2, **dadurch gekennzeichnet, dass** bei einer Verschiebung des ersten Hohlrohrs (10) und des zweiten Hohlrohrs (11) in Längsrichtung des Schafts (1) die erste Dichtung (19) auf der Innenfläche (18) des Schaftgehäuses (5) und/oder auf der Außenfläche des ersten Hohlrohrs (10) und die zweite Dichtung (25) auf der Innenfläche des ersten Hohlrohrs (10) und/oder auf der Außenfläche des zweiten Hohlrohrs (11) abdichtend gleitet.

4. Medizinisches Instrument (50) nach einem der vorhergehenden Ansprüche, **dadurch gekennzeichnet, dass** die erste und die zweite Dichtung (19, 25) mit dem ersten Hohlrohr (10) verbunden sind.

5. Medizinisches Instrument (50) nach einem der vorhergehenden Ansprüche, **dadurch gekennzeichnet, dass** das erste Hohlrohr (10) und das zweite Hohlrohr (11) aus dem Schaftgehäuse (5) in die Handhabe (54) hineinreichen.

6. Medizinisches Instrument (50) nach einem der vorhergehenden Ansprüche, **dadurch gekennzeichnet, dass** das zweite Übertragungselement aus dem Schaftgehäuse (5) in die Handhabe (54) hineinreicht.

7. Medizinisches Instrument (50) nach einem der vorhergehenden Ansprüche, **dadurch gekennzeichnet, dass** das erste Übertragungselement mindestens eine Spülöffnung (17, 17') aufweist.

8. Medizinisches Instrument (50) nach einem der vorhergehenden Ansprüche, **dadurch gekennzeichnet, dass** zwischen einer Innenfläche des zweiten Hohlrohrs (11) und einer Außenfläche des zweiten Übertragungselements eine ringförmige dritte Dichtung (31) angeordnet ist.

9. Medizinisches Instrument (50) nach einem der vorhergehenden Ansprüche, **dadurch gekennzeichnet, dass** das zweite Übertragungselement als Zugstange ausgebildet ist.

10. Medizinisches Instrument (50) nach einem der Ansprüche 1 bis 8, **dadurch gekennzeichnet, dass** das zweite Übertragungselement als Innenrohr (26) ausgebildet ist, wobei innerhalb des Innenrohrs (26) ein langerstrecktes drittes Übertragungselement angeordnet ist.

11. Medizinisches Instrument (50) nach einem der vorhergehenden Ansprüche, **dadurch gekennzeichnet, dass** das erste Funktionselement der Werkzeuganordnung (51) als gegenüber einer Längsachse des Schafts (1) abwinkelbarer Biegeabschnitt (53) ausgebildet ist, wobei an einem distalen Ende des Biegeabschnitts (53) das zweite Funktionselement angeordnet ist.

12. Medizinisches Instrument (50) nach einem der vorhergehenden Ansprüche, **dadurch gekennzeichnet, dass** das zweite Funktionselement als Arbeitswerkzeug mit mindestens einem beweglichen Werkzeugelement ausgebildet ist, das durch Verschiebung des zweiten Übertragungselements in Längsrichtung des Schafts (1) betätigbar ist.

13. Medizinisches Instrument (50) nach einem der vorhergehenden Ansprüche, **dadurch gekennzeichnet, dass** das Arbeitswerkzeug durch Rotation des zweiten Übertragungselements um die Längsachse des Schafts (1) um eine Längsachse des Arbeitswerkzeugs drehbar ist.

14. Medizinisches Instrument (50) mit einem langerstreckten Schaft (1), einer an einem distalen Ende des Schafts (1) angeordneten Werkzeuganordnung (51) und einer an einem proximalen Ende des Schafts (1) angeordneten Handhabe (54) zur Betätigung der Werkzeuganordnung (51), wobei der Schaft (1) ein langerstrecktes Außenrohr (3) aufweist, innerhalb dessen ein erstes Übertragungselement zur Übertragung einer Betätigungsbewegung von der Handhabe (54) zur Werkzeuganordnung (51) zur Betätigung mindestens eines ersten Funktionselements der Werkzeuganordnung (51) angeordnet ist, wobei das erste Übertragungselement ein erstes Halbrohr (13, 36) und ein zweites Halbrohr (14, 37) umfasst, die zur Betätigung des mindestens einen ersten Funktionselements gegeneinander in einer Längsrichtung des Schafts (1) verschiebbar sind, und wobei in einem von dem ersten Halbrohr (13, 36) und dem zweiten Halbrohr (14, 37) gebildeten längs erstreckten Hohlraum ein zweites Übertragungselement zur Übertragung einer Betätigungsbewegung von der Handhabe (54) zur Werkzeuganordnung (51) zur Betätigung mindestens eines zweiten Funktionselements der Werkzeuganordnung (51) angeordnet ist, **dadurch gekennzeichnet, dass** in einem proximalen Endabschnitt (4) des Schafts (1) ein gegenüber dem Schaft (1) erweitertes Schaftgehäuse (5) angeordnet ist, das mit dem Außenrohr (3) verbunden ist, und dass sich das erste Halbrohr (36) und das zweite Halbrohr (37) durch das Schaftgehäuse (5) hindurch erstrecken, wobei zwischen einer Innenfläche (18) des Schaftgehäuses (5) und den Außenflächen des ersten Halbrohrs (36) und des zweiten Halbrohrs (37) eine ringförmige Dichtung (38) angeordnet ist.

15. Medizinisches Instrument (50) nach Anspruch 14, **dadurch gekennzeichnet, dass** das erste Halbrohr (36) und das zweite Halbrohr (37) mit ihren offenen Längsseiten einander zugewandt sind, wobei das erste Halbrohr (36) und das zweite Halbrohr (37) zwischen ihren Längsschnittflächen (40, 40') jeweils einen Spalt (41) bilden, und dass zwischen einer Innenfläche des ersten Halbrohrs (36), einer Innenfläche des zweiten Halbrohrs (37) und einer Außenfläche des zweiten Übertragungselements eine ringförmige innere Dichtung (42) angeordnet ist und dass die zwischen der Innenfläche (18) des Schaftgehäuses (5) und den Außenflächen des ersten Halbrohrs (36) und des zweiten Halbrohrs (37) angeordnete ringförmige Dichtung (38) zwei einander gegenüberliegend nach innen ragende, sich axial erstreckende Stege (39, 39') aufweist, die jeweils in den Spalt (41) hineinragen und dichtend an den Längsschnittflächen (40, 40') des ersten Halbrohrs (36) und des zweiten Halbrohrs (37) sowie an der inneren Dichtung (42) anliegen.

## Claims

1. Medical instrument (50) having an elongate shank (1), a tool arrangement (51) arranged at a distal end of the shank (1) and a handle (54), which is arranged at a proximal end of the shank (1), for operating the tool arrangement (51), wherein the shank (1) has an elongate outer tube (3), within which a first transmission element for transmitting an operating movement from the handle (54) to the tool arrangement (51) in order to operate at least one first functional element of the tool arrangement (51) is arranged, wherein the first transmission element comprises a first half tube (13, 36) and a second half tube (14, 37), which half tubes are mutually displaceable in a longitudinal direction of the shank (1) in order to operate the at least one first functional element, and wherein a second transmission element for transmitting an operating movement from the handle (54) to the tool arrangement (51) in order to operate at least one second functional element of the tool arrangement (51) is arranged in an elongate cavity formed by the first half tube (13, 36) and the second half tube (14, 37), **characterized in that** a shank housing (5) which is enlarged in relation to the shank (1) and is connected to the outer tube (3) is arranged in a proximal end portion (4) of the shank (1), **in that** a first hollow tube (10) which is connected to the first half tube (13) is arranged within the shank housing (5), and **in that** a second hollow tube (11) which is connected to the second half tube (14) is arranged within the first hollow tube (10), wherein an annular first seal (19) is arranged between an inner surface (18) of the shank housing (5) and an outer surface of the first hollow tube (10) and an annular second seal (25) is arranged between an inner surface of the first hollow tube (10) and an outer surface of the second hollow tube (11).

2. Medical instrument (50) according to Claim 1, **characterized in that** the outer tube (3) and / or the shank housing (5) have / has an irrigation opening (8, 9, 9'), and **in that** the first seal (19) is arranged on the proximal side of the irrigation opening (8, 9, 9').

3. Medical instrument (50) according to Claim 1 or 2, **characterized in that**, when the first hollow tube (10) and the second hollow tube (11) are displaced in the longitudinal direction of the shank (1), the first seal (19) slides in a sealing manner on the inner surface (18) of the shank housing (5) and / or on the outer surface of the first hollow tube (10), and the second seal (25) slides in a sealing manner on the inner surface of the first hollow tube (10) and / or on the outer surface of the second hollow tube (11).

4. Medical instrument (50) according to one of the preceding claims, **characterized in that** the first and the second seal (19, 25) are connected to the first hollow tube (10).

5. Medical instrument (50) according to one of the preceding claims, **characterized in that** the first hollow tube (10) and the second hollow tube (11) extend out of the shank housing (5) into the handle (54).

6. Medical instrument (50) according to one of the preceding claims, **characterized in that** the second transmission element extends out of the shank housing (5) into the handle (54).

7. Medical instrument (50) according to one of the preceding claims, **characterized in that** the first transmission element has at least one irrigation opening (17, 17').

8. Medical instrument (50) according to one of the preceding claims, **characterized in that** an annular third seal (31) is arranged between an inner surface of the second hollow tube (11) and an outer surface of the second transmission element.

9. Medical instrument (50) according to one of the preceding claims, **characterized in that** the second transmission element is designed as a tension rod.

10. Medical instrument (50) according to one of Claims 1 to 8, **characterized in that** the second transmission element is designed as an inner tube (26), wherein an elongate third transmission element is arranged within the inner tube (26).

11. Medical instrument (50) according to one of the preceding claims, **characterized in that** the first functional element of the tool arrangement (51) is designed as a bending portion (53) which can be angled in relation to a longitudinal axis of the shank (1), wherein the second functional element is arranged at a distal end of the bending portion (53).

12. Medical instrument (50) according to one of the preceding claims, **characterized in that** the second functional element is designed as a working tool with at least one movable tool element which is operable by displacement of the second transmission element in the longitudinal direction of the shank (1).

13. Medical instrument (50) according to one of the preceding claims, **characterized in that** the working tool is rotatable about a longitudinal axis of the working tool by rotation of the second transmission element about the longitudinal axis of the shank (1).

14. Medical instrument (50) having an elongate shank (1), a tool arrangement (51) arranged at a distal end of the shank (1) and a handle (54), which is arranged at a proximal end of the shank (1), for operating the tool arrangement (51), wherein the shank (1) has an elongate outer tube (3), within which a first transmission element for transmitting an operating movement from the handle (54) to the tool arrangement (51) in order to operate at least one first functional element of the tool arrangement (51) is arranged, wherein the first transmission element comprises a first half tube (13, 36) and a second half tube (14, 37), which half tubes are mutually displaceable in a longitudinal direction of the shank (1) in order to operate the at least one first functional element, and wherein a second transmission element for transmitting an operating movement from the handle (54) to the tool arrangement (51) in order to operate at least one second functional element of the tool arrangement (51) is arranged in an elongate cavity formed by the first half tube (13, 36) and the second half tube (14, 37), **characterized in that** a shank housing (5) which is enlarged in relation to the shank (1) and is connected to the outer tube (3) is arranged in a proximal end portion (4) of the shank (1), and **in that** the first half tube (36) and the second half tube (37) extend through the shank housing (5), wherein an annular seal (38) is arranged between an inner surface (18) of the shank housing (5) and the outer surfaces of the first half tube (36) and of the second half tube (37).

15. Medical instrument (50) according to Claim 14, **characterized in that** the first half tube (36) and the second half tube (37) face each other with their open longitudinal sides, wherein the first half tube (36) and the second half tube (37) in each case form a gap (41) between their longitudinal sectional surfaces (40, 40'), and **in that** an annular inner seal (42) is arranged between an inner surface of the first half tube (36), an inner surface of the second half tube (37) and an outer surface of the second transmission element, and **in that** the annular seal (38) arranged between the inner surface (18) of the shank housing (5) and the outer surfaces of the first half tube (36) and of the second half tube (37) has two axially extending webs (39, 39') which protrude inwards opposite each other, in each case project into the gap (41) and lie in a sealing manner against the longitudinal sectional surfaces (40, 40') of the first half tube (36) and of the second half tube (37) and against the inner seal (42)

## Revendications

1. Instrument médical (50) comprenant une tige allongée (1), un agencement d'outil (51) disposé à une extrémité distale de la tige (1) et une manette (54) disposée à une extrémité proximale de la tige (1) pour actionner l'agencement d'outil (51), la tige (1) présentant un tube extérieur allongé (3), à l'intérieur duquel est disposé un premier élément de transmission pour transmettre un mouvement d'actionnement de la manette (54) à l'agencement d'outil (51) pour actionner au moins un premier élément fonctionnel de l'agencement d'outil (51), le premier élément de transmission comprenant un premier demi-tube (13, 36) et un deuxième demi-tube (14, 37), qui peuvent être déplacés l'un par rapport à l'autre dans une direction longitudinale de la tige (1) pour l'actionnement de l'au moins un premier élément fonctionnel, et un deuxième élément de transmission pour transmettre un mouvement d'actionnement de la manette (54) à l'agencement d'outil (51) pour l'actionnement d'au moins un deuxième élément fonctionnel de l'agencement d'outil (51) étant disposé dans une cavité s'étendant longitudinalement formée par le premier demi-tube (13, 36) et le deuxième demi-tube (14, 37), **caractérisé en ce qu'**un boîtier de tige (5) élargi par rapport à la tige (1) est disposé dans une portion d'extrémité proximale (4) de la tige (1), lequel est connecté au tube extérieur (3), **en ce qu'**un premier tube creux (10) est disposé à l'intérieur du boîtier de tige (5), lequel est connecté au premier demi-tube (13), et **en ce qu'**un deuxième tube creux (11) est disposé à l'intérieur du premier tube creux (10), lequel est connecté au deuxième demi-tube (14), un premier joint d'étanchéité de forme annulaire (19) étant disposé entre une surface intérieure (18) du boîtier de tige (5) et une surface extérieure du premier tube creux (10) et un deuxième joint d'étanchéité de forme annulaire (25) étant disposé entre une surface intérieure du premier tube creux (10) et une surface extérieure du deuxième tube creux (11).

2. Instrument médical (50) selon la revendication 1, **caractérisé en ce que** le tube extérieur (3) et / ou le boîtier de tige (5) présente une ouverture de rinçage (8, 9, 9') et **en ce que** le premier joint d'étanchéité (19) est disposé du côté proximal de l'ouverture de rinçage (8, 9, 9').

3. Instrument médical (50) selon la revendication 1 ou 2, **caractérisé en ce que** lors d'un déplacement du premier tube creux (10) et du deuxième tube creux (11) dans la direction longitudinale de la tige (1), le premier joint d'étanchéité (19) glisse de manière hermétique sur la surface intérieure (18) du boîtier de tige (5) et / ou sur la surface extérieure du premier tube creux (10) et le deuxième joint d'étanchéité (25) glisse de manière hermétique sur la surface intérieure du premier tube creux (10) et / ou sur la surface extérieure du deuxième tube creux (11).

4. Instrument médical (50) selon l'une quelconque des revendications précédentes, **caractérisé en ce que** le premier et le deuxième joint d'étanchéité (19, 25) sont connectés au premier tube creux (10).

5. Instrument médical (50) selon l'une quelconque des revendications précédentes, **caractérisé en ce que** le premier tube creux (10) et le deuxième tube creux (11) s'étendent hors du boîtier de tige (5) dans la manette (54).

6. Instrument médical (50) selon l'une quelconque des revendications précédentes, **caractérisé en ce que** le deuxième élément de transmission pénètre hors du boîtier de tige (5) dans la manette (54).

7. Instrument médical (50) selon l'une quelconque des revendications précédentes, **caractérisé en ce que** le premier élément de transmission présente au moins une ouverture de rinçage (17, 17').

8. Instrument médical (50) selon l'une quelconque des revendications précédentes, **caractérisé en ce qu'**entre une surface intérieure du deuxième tube creux (11) et une surface extérieure du deuxième élément de transmission est disposé un troisième joint d'étanchéité de forme annulaire (31).

9. Instrument médical (50) selon l'une quelconque des revendications précédentes, **caractérisé en ce que** le deuxième élément de transmission est réalisé sous forme de tige de traction.

10. Instrument médical (50) selon l'une quelconque des revendications 1 à 8, **caractérisé en ce que** le deuxième élément de transmission est réalisé sous forme de tube interne (26), un troisième élément de transmission allongé étant disposé à l'intérieur du tube interne (26).

11. Instrument médical (50) selon l'une quelconque des revendications précédentes, **caractérisé en ce que** le premier élément fonctionnel de l'agencement d'outil (51) est réalisé sous forme de portion de flexion (53) pouvant être coudée par rapport à un axe longitudinal de la tige (1), le deuxième élément fonctionnel étant disposé au niveau d'une extrémité distale de la portion de flexion (53).

12. Instrument médical (50) selon l'une quelconque des revendications précédentes, **caractérisé en ce que** le deuxième élément fonctionnel est réalisé sous forme d'outil de travail comprenant au moins un élément d'outil mobile, qui peut être actionné par déplacement du deuxième élément de transmission dans la direction longitudinale de la tige (1).

13. Instrument médical (50) selon l'une quelconque des revendications précédentes, **caractérisé en ce que** l'outil de travail peut être tourné par rotation du deuxième élément de transmission autour de l'axe longitudinal de la tige (1) autour d'un axe longitudinal de l'outil de travail.

14. Instrument médical (50) comprenant une tige allongée (1), un agencement d'outil (51) disposé à une extrémité distale de la tige (1) et une manette (54) disposée à une extrémité proximale de la tige (1) pour actionner l'agencement d'outil (51), la tige (1) présentant un tube extérieur allongé (3) à l'intérieur duquel est disposé un premier élément de transmission pour la transmission d'un mouvement d'actionnement de la manette (54) à l'agencement d'outil (51) pour l'actionnement d'au moins un premier élément fonctionnel de l'agencement d'outil (51), le premier élément de transmission présentant un premier demi-tube (13, 36) et un deuxième demi-tube (14 37), qui peuvent être déplacés l'un par rapport à l'autre dans une direction longitudinale de la tige (1) pour l'actionnement de l'au moins un premier élément fonctionnel, et un deuxième élément de transmission pour la transmission d'un mouvement d'actionnement de la manette (54) à l'agencement d'outil (51) pour l'actionnement d'au moins un deuxième élément fonctionnel de l'agencement d'outil (51) étant disposé dans une cavité s'étendant longitudinalement formée par le premier demi-tube (13, 36) et le deuxième demi-tube (14, 37), **caractérisé en ce qu'**un boîtier de tige (5) élargi par rapport à la tige (1) est disposé dans une portion d'extrémité proximale (4) de la tige (1), lequel est connecté au tube extérieur (3) et **en ce que** le premier demi-tube (36) et le deuxième demi-tube (37) s'étendent à travers le boîtier de tige (5), un joint d'étanchéité de forme annulaire (38) étant disposé entre une surface intérieure (18) du boîtier de tige (5) et les surfaces extérieures du premier demi-tube (36) et du deuxième demi-tube (37).

15. Instrument médical (50) selon la revendication 14, **caractérisé en ce que** le premier demi-tube (36) et le deuxième demi-tube (37) sont tournés l'un vers l'autre avec leurs côtés longitudinaux ouverts, le premier demi-tube (36) et le deuxième demi-tube (37) formant entre leurs surfaces en coupe longitudinale (40, 40') à chaque fois une fente (41) et **en ce qu'**entre une surface intérieure du premier demi-tube (36), une surface intérieure du deuxième demi-tube (37) et une surface extérieure du deuxième élément de transmission est disposé un joint d'étanchéité interne de forme annulaire (42) et **en ce que** le joint d'étanchéité de forme annulaire (38) disposé entre la surface intérieure (18) du boîtier de tige (5) et les surfaces extérieures du premier demi-tube (36) et du deuxième demi-tube (37) présente deux nervures (39, 39') s'étendant axialement, faisant saillie vers l'intérieur, opposées l'une à l'autre, qui pénètrent à chaque fois dans la fente (41) et qui s'appliquent hermétiquement contre les surfaces en coupe longitudinale (40, 40') du premier demi-tube (36) et du deuxième demi-tube (37) ainsi que contre le joint d'étanchéité interne (42).
